(19) Europäisches Patentamt
European Patent Office
Office européen des brevets

(11) EP 4 176 731 A1

(12) **EUROPEAN PATENT APPLICATION**
published in accordance with Art. 153(4) EPC

(43) Date of publication:
**10.05.2023 Bulletin 2023/19**

(21) Application number: **21854145.6**

(22) Date of filing: **02.08.2021**

(51) International Patent Classification (IPC):
***A23L 3/3544*** *(2006.01)* ***A61P 31/00*** *(2006.01)*
***A23L 27/00*** *(2016.01)* ***A23L 27/50*** *(2016.01)*
***A01P 3/00*** *(2006.01)* ***A01N 43/16*** *(2006.01)*
***A61K 31/343*** *(2006.01)* ***A61K 31/352*** *(2006.01)*
***A61K 31/353*** *(2006.01)* ***A61K 31/37*** *(2006.01)*

(52) Cooperative Patent Classification (CPC):
**A01N 43/16; A01P 3/00; A23L 3/3544; A23L 27/00; A23L 27/50; A61K 31/343; A61K 31/352; A61K 31/353; A61K 31/37; A61P 31/00**

(86) International application number:
**PCT/JP2021/028606**

(87) International publication number:
**WO 2022/030443 (10.02.2022 Gazette 2022/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.08.2020 JP 2020135109**

(71) Applicant: **Kikkoman Corporation**
**Noda-shi, Chiba 278-8601 (JP)**

(72) Inventors:
- **SHIMABE Eiji**
**Noda-shi, Chiba 278-8601 (JP)**
- **UCHIDA Riichiro**
**Noda-shi, Chiba 278-8601 (JP)**
- **KOBAYASHI Sachiko**
**Noda-shi, Chiba 278-8601 (JP)**
- **MARUSHIMA Kazuya**
**Noda-shi, Chiba 278-8601 (JP)**
- **ENDO Yoshikazu**
**Noda-shi, Chiba 278-8601 (JP)**
- **OHIRA Takuya**
**Noda-shi, Chiba 278-8601 (JP)**

(74) Representative: **Mewburn Ellis LLP**
**Aurora Building**
**Counterslip**
**Bristol BS1 6BX (GB)**

(54) **FOOD/BEVERAGE ARTICLE**

(57) A food/beverage article, comprising:
one or more compounds represented by formula (I):

[Chemical Formula 1]

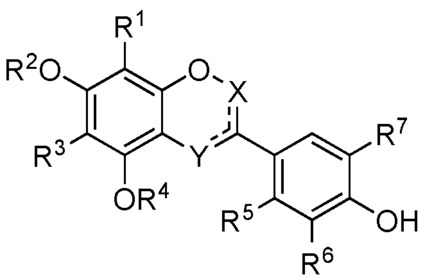

(I)

wherein, $R^1$ and $R^3$ each independently represent a hydrogen atom or a $C_{2-6}$ alkenyl group, $R^2$ and $R^4$ each independently represent a hydrogen atom or a $C_{1-4}$ alkyl group, $R^5$ and $R^7$ each independently represent a hydroxyl group or a hydrogen atom or a hydroxyl group, $R^6$ represents a hydrogen atom or a $C_{2-6}$ alkenyl group, X represents a direct bond, $-CH_2-$, -CH=, or -C(=O)-, and Y represents $-CH_2-$, -CH=, or -C(=O)-, wherein the total of the concentrations of the compounds is 1 ppm or more.



Processed by Luminess, 75001 PARIS (FR)

## Description

### Technical Field

The present invention relates to a food/beverage article containing an oxygen-containing heterocyclic compound.

### Background Art

Low fungus-resistant food/beverage articles such as low-salt soy sauce and ready-to-use Japanese seasoned soup stock containing a small amount of salt have been distributed due to an increase in the health orientation of consumers and the like in recent years. If such low fungus-resistant food/beverage articles are contaminated with lactic acid bacteria, the food/beverage articles are deteriorated, resulting in, for example, pH change due to the generation of lactic acid or the decarboxylation of amino acids, and the deformation of the containers due to the generation of carbonic acid gas, and thus the qualities of products are markedly spoiled disadvantageously (for example, Non Patent Literature 1). Also, if low fungus-resistant food/beverage articles are contaminated with flat sour bacteria, the contents are acidified due to acid production and the qualities of products are markedly spoiled disadvantageously (for example, Non Patent Literature 2).

### Citation List

### Non Patent Literature

Non Patent Literature 1
JOURNAL OF THE BREWING SOCIETY OF JAPAN, 2008, Vol. 103, No. 2, p. 94-99
Non Patent Literature 2
B. coagulans, edited by Tateo Fujii, Fundamentals of Food Microbiology, KODANSHA LTD., 2015, p. 15

### Summary of Invention

### Technical Problem

Examples of means for suppressing the deterioration of food/beverage articles due to contamination with lactic acid bacteria and/or flat sour bacteria include synthetic preservative addition, an increase in the amount of salt and/or alcohol, and acidification by reducing the pH. It cannot, however, be said that any method described above is desirable in view of health orientation and the savor of food.

An object of the present invention is to provide a food/beverage article that is unlikely to be deteriorated due to lactic acid bacteria and/or flat sour bacteria contamination without depending on these methods.

### Solution to Problem

As a result of earnest studies to solve the above-mentioned problem, the present inventors have consequently found that an oxygen-containing heterocyclic compound such as licoricidin represented by the following formula (I) exhibits antimicrobial activity to lactic acid bacteria and/or flat sour bacteria, and completed the present invention based on the finding.

Specifically, the present invention provides a food/beverage article comprising: one or more compounds represented by the following formula (I):

[Chemical Formula 1]

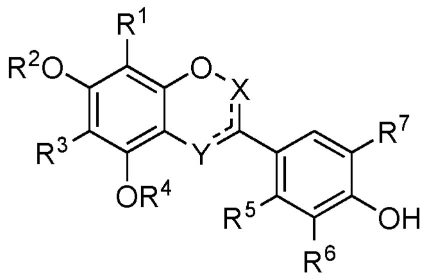

(I)

wherein, $R^1$ and $R^3$ each independently represent a hydrogen atom or a $C_{2-6}$ alkenyl group, $R^2$ and $R^4$ each independently represent a hydrogen atom or a $C_{1-4}$ alkyl group, $R^5$ and $R^7$ each independently represent a hydrogen atom or a hydroxyl group, $R^6$ represents a hydrogen atom or a $C_{2-6}$ alkenyl group, X represents a direct bond, $-CH_2-$, -CH=, or -C(=O)-, Y represents $-CH_2-$, -CH=, or -C(=O)-, and a bond represented by the following formula:

[Chemical Formula 2] 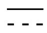

represents a single bond or a double bond (hereinafter also referred to as a "compound (I)"), wherein the total of the concentrations of the compounds is 1 ppm or more. Since the food/beverage article of the present invention contains the compound (I) at 1 ppm or more, the food/beverage article exhibits antimicrobial activity to lactic acid bacteria and/or flat sour bacteria, and is unlikely to be deteriorated due to lactic acid bacteria and/or flat sour bacteria contamination.

It is preferable that the compound (I) be at least one selected from the group consisting of licoricidin, gancaonin I, 8-(y,y-dimethylallyl)-wighteone, glycycoumarin, glyasperin C, glycyrin, isoangustone A, and licoarylcoumarin.

The food/beverage article of the present invention may be liquid seasoning or food. The above-mentioned liquid seasoning may be soy sauce, soup stock, Japanese seasoned soup stock, sauce, dressing, or cooking vinegar, and the above-mentioned food may be lightly-pickled vegetables.

The present invention provides an antimicrobial agent to lactic acid bacteria and/or flat sour bacteria, comprising: the compound (I) as an active ingredient.

The present invention provides a method for suppressing the proliferation of lactic acid bacteria and/or flat sour bacteria in the food/beverage article, comprising: adjusting the total of the concentrations of the compounds (I) in the food/beverage article to 1 ppm or more.

## Advantageous Effects of Invention

According to the present invention, a food/beverage article is unlikely to be deteriorated due to lactic acid bacteria and/or flat sour bacteria contamination can be provided. According to the present invention, a novel antimicrobial agent to lactic acid bacteria and/or flat sour bacteria can be provided. According to the present invention, a method for suppressing the proliferation of lactic acid bacteria and/or flat sour bacteria in the food/beverage article can be provided.

## Description of Embodiments

Hereinafter, preferred embodiments of the present invention will be described in detail. However, the present invention is not limited to the following embodiments.

A food/beverage article according to the present embodiment contains a compound represented by the following formula (I) at 1 ppm or more:

[Chemical Formula 3]

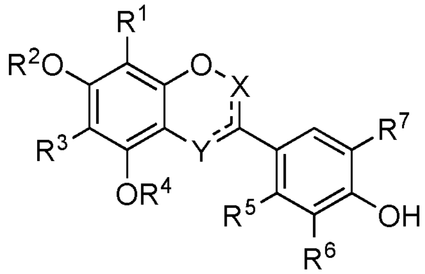

(I)

wherein, $R^1$ and $R^3$ each independently represent a hydrogen atom or a $C_{3-6}$ alkenyl group, $R^2$ and $R^4$ each independently represent a hydrogen atom or a $C_{1-4}$ alkyl group, $R^5$ and $R^7$ each independently represent a hydrogen atom or a hydroxyl group, $R^6$ represents a hydrogen atom or a $C_{2-6}$ alkenyl group, X represents a direct bond, $-CH_2-$, -CH=, or -C(=O)-, Y represents $-CH_2-$, -CH=, or -C(=O)-, and a bond represented by the following formula:

[Chemical Formula 4] 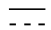

represents a single bond or a double bond.

The "$C_{1-4}$ alkyl group" used herein means a linear or branched alkyl group having 1 to 4 carbon atoms. Examples of the $C_{1-4}$ alkyl group include a methyl group, an ethyl group, a propyl group, an isopropyl group, a butyl group, an isobutyl group, a sec-butyl group, and a tert-butyl group.

The "$C_{2-6}$ alkenyl group" used herein means a linear or branched alkenyl group having 2 to 6 carbon atoms. Examples of the $C_{2-6}$ alkenyl group include a vinyl group, a propen-1-yl group, a propen-2-yl group, a 1-butenyl group, a 2-butenyl group, a 3-butenyl group, a 1-methyl-1-propenyl group, a 2-methyl-1-propenyl group, a 1-pentenyl group, a 2-pentenyl group, a 3-pentenyl group, a 4-pentenyl group, a 5-pentenyl group, a 1-methyl-1-butenyl group, a 2-methyl-1-butenyl group, a 3-methyl-1-butenyl group, a 4-methyl-1-butenyl group, a 1-methyl-2-butenyl group, a 2-methyl-2-butenyl group, a 3-methyl-2-butenyl group, a 4-methyl-2-butenyl group, a 1-methyl-3-butenyl group, a 2-methyl-3-butenyl group, a 3-methyl-3-butenyl group, a 4-methyl-3-butenyl group, a 1,2-dimethyl-1-propenyl group, a 1,1-dimethyl-2-propenyl group, a 1-hexenyl group, a 2-hexenyl group, a 3-hexenyl group, a 4-hexenyl group, a 5-hexenyl group, and a 6-hexenyl group.

In above-mentioned formula (I), $R^1$ is preferably a hydrogen atom, a 3-methyl-2-butenyl group, or a 1,1-dimethyl-2-propenyl group.

In above-mentioned formula (I), $R^2$ is preferably a hydrogen atom or a methyl group.

In above-mentioned formula (I), $R^3$ is preferably a hydrogen atom or a 3-methyl-2-butenyl group.

In above-mentioned formula (I), $R^4$ is preferably a hydrogen atom or a methyl group.

In above-mentioned formula (I), $R^6$ is preferably a hydrogen atom or a 3-methyl-2-butenyl group.

The compound (I) may have isomers such as stereoisomers and tautomers. Those isomers are also included in the scope of the present invention.

Specific examples of the compound (I) include licoricidin, gancaonin I, 8-(γ,γ-dimethylallyl)-wighteone, glycycoumarin, glyasperin C, glycyrin, isoangustone A, and licoarylcoumarin.

Licoricidin is also called 4-[(R)-7-hydroxy-5-methoxy-6-(3-methyl-2-butenyl)chroman-3-yl]-2-( 3-methyl-2-butenyl)-1,3-benzenediol, and is a known compound represented by the following formula:

[Chemical Formula 5]

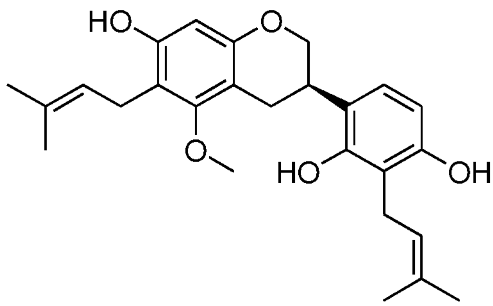

Gancaonin I is also called 5-(3-methyl-2-butenyl)-2-(2,4-dihydroxyphenyl)-4,6-dimethoxybenzofu ran, and is a known compound represented by the following formula:

[Chemical Formula 6]

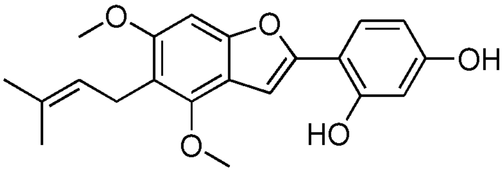

8-(y,y-Dimethylallyl)-wighteone is also called 6,8-bis(3-methyl-2-butenyl)-3-(4-hydroxyphenyl)-5,7-dihydroxy-4H-1-benzopyran-4-one, and is a known compound represented by the following formula:

[Chemical Formula 7]

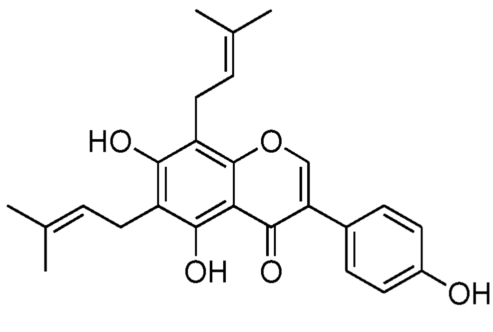

Glycycoumarin is also called 3-(2,4-dihydroxyphenyl)-7-hydroxy-5-methoxy-6-(3-methyl-2-butenyl)-2H-1-benzopyran-2-one, and is a known compound represented by the following formula:

[Chemical Formula 8]

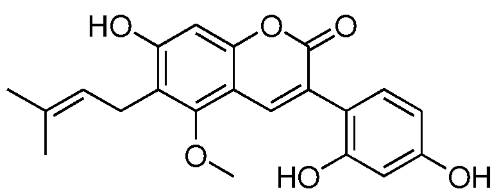

Glyasperin C is also called (3R)-3β(2,4-dihydroxyphenyl)-5-methoxy-6-(3-methyl-2-butenyl)-3,4-d ihydro-2H-1-benzopyran-7-ol, and is a known compound the following formula:

[Chemical Formula 9]

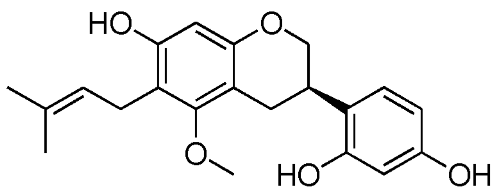

Glycyrin is also called 3-(2,4-dihydroxyphenyl)-5,7-dimethoxy-6-(3-methyl-2-butenyl)-2H-1-b enzopyran-2-one, and is a known compound represented by the following formula:

[Chemical Formula 10]

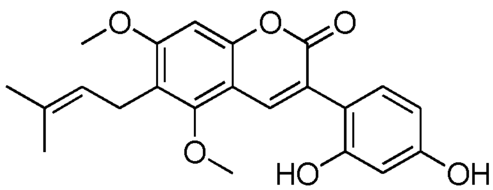

Isoangustone A is also called 3-[3,4-dihydroxy-5-(3-methyl-2-butenyl)phenyl]-5,7-dihydroxy-6-(3-m ethyl-2-butenyl)-4H-chromen-4-one, and is a known compound represented by the following formula:

[Chemical Formula 11]

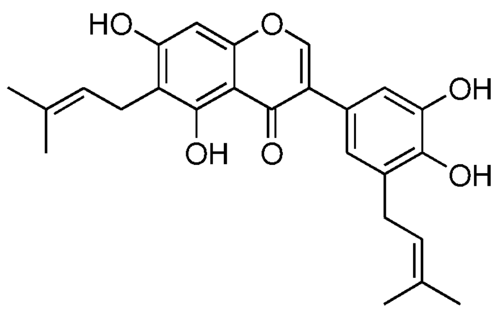

Licoarylcoumarin is also called 3-(2,4-dihydroxyphenyl)-7-hydroxy-5-methoxy-8-(2-methyl-3-butene-2 -yl)coumarin, and is a known compound represented by the following formula:

[Chemical Formula 12]

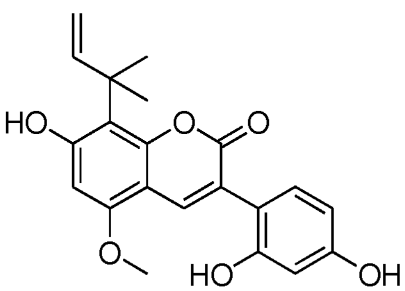

The compound (I) may be synthesized by a method known to those skilled in the art, or may be a commercially available product. Licoricidin, gancaonin I, 8-(γ,γ-dimethylallyl)-wighteone, glycycoumarin, glyasperin C, glycyrin, isoangustone A, and licoarylcoumarin are known to be compounds contained in licorice oily extract. Accordingly, in the food/beverage article according to the present embodiment, licorice oily extract containing the compound (I) can also be used as it is, or the compound (I) contained in licorice oily extract can be isolated therefrom before use.

The food/beverage article according to the present embodiment may contain only one compound (I), or may contain two or more compounds (I). When the food/beverage article according to the present embodiment contains two or more compounds (I), the concentration of the compounds (I) means the total concentration of the concentrations of the compounds.

In the food/beverage article according to the present embodiment, the concentration of the compound (I) is 1 ppm or more, in terms of the lower limit. This enables suppression of the deterioration of the food/beverage article due to lactic acid bacteria and/or flat sour bacteria contamination. The concentration of the compound (I) can be suitably set to the range of 1 ppm or more according to the use of the food/beverage article, the salt concentration, the alcohol concentration, the pH, and the like, and the concentration of the compound (I) may be, for example, 5 ppm or more, may be 10 ppm or more, may be 25 ppm or more, or may be 50 ppm or more in view of further enhancing the antimicrobial activity to lactic acid bacteria and/or flat sour bacteria.

In the food/beverage article according to the present embodiment, the upper limit of the concentration of the compound (I) is not particularly limited, and the concentration of the compound (I) may be, for example, 10000 ppm or less in view of dissolving the compound (I) in the target food/beverage article adequately.

For example, the concentration of the compound (I) can be measured by liquid chromatography and tandem mass spectrometry (LC/MS/MS).

The food/beverage article according to the present embodiment may be any food/beverage article without particular limitation, as long as it is a food/beverage article containing the compound (I) at 1 ppm or more. Examples of the food/beverage article according to the present embodiment include liquid seasonings such as soy sauce, soup stock, Japanese seasoned soup stock, sauce, soup, Worcester sauce, and dressing; semisolid seasonings such as miso and mayonnaise; pickled Japanese plums; pickles; and delicatessen. It is preferable that the food/beverage article according to the present embodiment be liquid seasoning or food, since the deterioration due to lactic acid bacteria and/or flat sour bacteria contamination can be further suppressed. Soy sauce, soup stock, Japanese seasoned soup stock, sauce, dressing, or cooking vinegar is preferable among the liquid seasonings. Lightly-pickled vegetables are preferable among the foods.

Although the salt concentration of the food/beverage article according to the present embodiment is not particularly limited, the salt concentration may be, for example, 12% (w/v) or less, 8% (w/v) or less, 4% (w/v) or less, or 0% (w/v) (unsalted), in view of enhancing the antimicrobial activity to lactic acid bacteria and/or flat sour bacteria without increasing the amount of salt.

For example, the salt concentration can be measured by a known method such as potentiometric titration, the Mohr method, or atomic absorption spectrophotometry.

Although the alcohol concentration of the food/beverage article according to the present embodiment is not particularly limited, the alcohol concentration may be, for example, 10% (v/v) or less, 5% (v/v) or less, or 0% (v/v), in view of enhancing the antimicrobial activity to lactic acid bacteria and/or flat sour bacteria without increasing the amount of alcohol.

For example, the alcohol concentration can be measured by gas chromatography.

Although the pH of the food/beverage article according to the present embodiment is not particularly limited, the pH may be, for example, 3.0 or more, 4.0 or more, or 4.2 or more, and may be 7.0 or less, 6.0 or less, or 5.8 or less, since the savor of the food/beverage article is good.

The food/beverage articles according to the present embodiment can be produced by, for example, adding licorice oily extract containing the compound (I) or the compound (I) to a food/beverage article as a base (preferably liquid seasoning or food) to a concentration of 1 ppm or more, and, if necessary, diluting or concentrating (e.g., deaeration, heating, drying, heating and deaeration at reduced pressure) the resultant and adding various additives, for example.

As confirmed in the following Examples, the compound (I) exhibits antimicrobial action on lactic acid bacteria and/or flat sour bacteria. Therefore, one aspect of the present invention provides an antimicrobial agent to lactic acid bacteria and/or flat sour bacteria containing the compound (I) as an active ingredient.

The antimicrobial agent according to the present embodiment can be used for various harm lactic acid bacteria (lactic acid bacteria encompassed by, for example, *Lactobacillus, Streptococcus,* and *Lactococcus*) and flat sour bacteria (for example, *Bacillus coagulans*).

The antimicrobial agent according to the present embodiment may be any of forms such as a solid (for example, powder), liquid (for example, a solution or a suspension), and a paste. The antimicrobial agent may be any dosage form such as powder, granules, a tablet, a capsule, a solution, a suspension, or a syrup. The above-mentioned various formulations can be prepared by mixing Licoricidin with additives (a vehicle, a binder, a lubricant, a disintegrator, an

emulsifier, a surfactant, a base, a solubilizer, a suspending agent, and the like) and molding the resulting mixture, as needed.

The antimicrobial agent according to the present embodiment can be used by adding it to any food/beverage article. Examples of such a food/beverage article include liquid seasonings such as soy sauce, soup stock, Japanese seasoned soup stock, sauce, soup, Worcestershire sauce, dressing, and cooking vinegar; semisolid seasonings such as miso and mayonnaise; pickled Japanese plums; pickles; and delicatessen.

As confirmed in the following Examples, the compound (I) exhibits the action of suppressing the proliferation of lactic acid bacteria and/or flat sour bacteria. Therefore, one aspect of the present invention provides a method for suppressing the proliferation of lactic acid bacteria and/or flat sour bacteria in a food/beverage article, comprising: adjusting the total of the concentrations of the compounds (I) in the food/beverage article to 1 ppm or more.

## Examples

Hereinafter, the present invention will be described by way of Examples more specifically. However, the present invention is not limited by the following Examples.

[Test Example 1: antimicrobial action of edible plant extracts on lactic acid bacteria]

(Preparation of extract)

To pulverized leaves, stems, or roots of commercially available edible plants (described in Tables 1 and 2) was added sterilized water in the same amount as that of the pulverized material, and the mixture was stirred and mixed with a vortex and left to stand at room temperature for 1 hour. To this mixture was added ethanol in 3 times the volume of the sterilized water, and extraction was performed in an incubator under the conditions of 50°C and 120 rpm (reciprocal shaking) for 3 hours to obtain 75% ethanol extraction liquid. This extraction liquid was centrifuged at 3000 rpm for 10 minutes to obtain extracts.

(Preparation of soy sauce)

Commercially available low-salt soy sauce (produced by Kikkoman Corporation) was two-fold diluted with sterilized water. The pH of the dilution was adjusted to 4.8 with sodium hydroxide, and the mixture was heat-disinfected to prepare test low-salt soy sauce.

(Evaluation of antimicrobial action)

First, 11.3 mL of test low-salt soy sauce, 0.7 mL of the extract, and lactic acid bacteria *(Lactobacillus rennini* and *Lactobacillus acidipiscis*) were added to a test tube to a final concentration of around $10^5$ cfu/mL, and a SILICOSEN was put in the test tube. The mixture was left to stand at 30°C for anaerobic culture. The proliferation of lactic acid bacteria produces precipitate of bacterial cells; accordingly, the precipitate produced on the bottom of the test tube was observed over time. The number of days until the generation of the precipitation started in the test, to which the extract was added, was compared with that in a control (mixture to which an aqueous 75% ethanol solution was added instead of the extract), to evaluate the antimicrobial action. The results are shown in Tables 1 and 2.

[Table 1]

| Edible plant | Difference in the number of days until precipitation | Edible plant | Difference in the number of days until precipitation | Edible plant | Difference in the number of days until precipitation |
|---|---|---|---|---|---|
| Kachiwari (pumpkin) | No difference | White cucumber (cucumber) | No difference | Borage | No difference |
| Ogato (pumpkin) | No difference | Spontaneous odama (tomato) | No difference | Coriander | No difference |
| Kamikochi (cucumber) | No difference | Bonita (tomato) | No difference | Artichoke | No difference |

(continued)

| Edible plant | Difference in the number of days until precipitation | Edible plant | Difference in the number of days until precipitation | Edible plant | Difference in the number of days until precipitation |
|---|---|---|---|---|---|
| Bateshirazu (cucumber) | No difference | Broccoli | No difference | Crimson clover | No difference |
| Myoko (tomato) | No difference | Red cabbage | No difference | Nigella | No difference |
| Heart-heart (tomato) | No difference | White radish sprout | No difference | Nagaokana | No difference |
| Chako (cherry tomato) | No difference | Tsumamina | No difference | Osakashirona | No difference |
| Brazil cook (cooking tomato) | No difference | Turnip | No difference | Hiroshimana | No difference |
| Shin-etsu mizunasu | No difference | Buckwheat | No difference | Katsuona | No difference |
| Native aonasu | No difference | Water spinach | Difference of less than 3 days | Kumamotokyona | No difference |
| Manganji-amato | No difference | Rocket | Difference of less than 3 days | Yamashiona | Difference of 3 to 6 days |
| Kurokodama-suika | No difference | Basil | No difference | Mibuna | No difference |
| Tenchoha (makuwa) | No difference | Mustard | Difference of less than 3 days | Bashona | No difference |
| Chikumano-gosun (carrot) | Difference of less than 3 days | White sesame | No difference | Kobutakana | Difference of 3 to 6 days |
| Eruwan (leaf lettuce) | No difference | Nozawana | No difference | Kalonji/ Nigella | No difference |
| Erugo (butterhead lettuce) | No difference | Green pea | No difference | Kumquat seed | No difference |
| Shimamura kidney bean | No difference | Black mappe | No difference | Bitter melon seed | No difference |
| Koshigaya kidney bean | No difference | Alphalpha | No difference | Pomegranate seed | No difference |
| Mochitto-corn | No difference | Perilla | No difference | Prune seed | No difference |
| Kiso purple turnip | No difference | Endive | No difference | Coriander | No difference |
| Shinkaiaona (komatsuna) | No difference | Kale | No difference | Basil | Difference of less than 3 days |
| Yasaiegoma | No difference | Soup celery | Difference of 7 days or more | Cayenne pepper | No difference |
| Fukkura (ruccola) | Difference of 3 to 6 days | Fennel | Difference of less than 3 days | Parsley | No difference |
| Curled mallow | No difference | Mike-takana | No difference | Habanero | No difference |

(continued)

| Edible plant | Difference in the number of days until precipitation | Edible plant | Difference in the number of days until precipitation | Edible plant | Difference in the number of days until precipitation |
|---|---|---|---|---|---|
| Kawashima kakina | No difference | Green mappe | No difference | Paprika | No difference |
| Amaranth | No difference | Bean sprout | No difference | Fenugreek | No difference |
| Black-eyed pea | No difference | Papaya | No difference | Fennel | No difference |
| Spontaneous green pepper | No difference | Koryan-mai mochi | No difference | Bhut Jolokia | No difference |
| Cherry tomato | No difference | Red rice | No difference | Fried onion | No difference |
| Miyashige (daikon) | No difference | Black rice | No difference | Marjoram | No difference |
| Radish | No difference | Linseed | No difference | Onion powder | No difference |
| Beet | No difference | Amaranth | No difference | Red pepper/ chili | No difference |
| Green coin (tatsoi) | No difference | Perilla | No difference | Lemon grass | Difference of less than 3 days |
| Anton (squash) | No difference | Quinoa | No difference | Kiwi fruit seed extract | No difference |
| Y-star (squash) | No difference | Poppy seed | No difference | Cacao extract | No difference |
| Celery | Difference of less than 3 days | Chicory | Difference of less than 3 days | Perilla seed extract | No difference |
| White Lisbon (long Welsh onion) | No difference | Winter melon (seed) | Difference of less than 3 days | Chinese chive seed extract | No difference |
| Leek (Welsh onion) | No difference | Pino green | No difference | Broccoli sprout | No difference |
| Chinese chive | No difference | Green spinach | No difference | Yuzu seed extract | No difference |
| Coriander | No difference | Red beet | No difference | Kanka extract | Difference of 3 to 6 days |
| Italian parsley | Difference of less than 3 days | Red Asian mustard | Difference of 3 to 6 days | Japanese butterbur extra | No difference |
| Rocket (rucola) | Difference of less than 3 days | Tatsoi | No difference | Buckwheat leaf extract | No difference |
| Rocky wild (rucola) | Difference of less than 3 days | Choy sum | No difference | Satsuma orange extract | No difference |
| Sage | No difference | Aonaga daikon | No difference | Fermented rice germ extract | No difference |
| Time | No difference | Koshin daikon | No difference | Black rice extract | No difference |
| Sweet basil | No difference | Benimaru daikon | No difference | Red rice extract | No difference |
| Sweet marjoram | No difference | Napa cabbage | No difference | Papaya peel | No difference |

(continued)

| Edible plant | Difference in the number of days until precipitation | Edible plant | Difference in the number of days until precipitation | Edible plant | Difference in the number of days until precipitation |
|---|---|---|---|---|---|
| Lemon basil | No difference | Purple-stem mustard | No difference | Dragon fruit peel | No difference |
| Lemon balm | No difference | Shiroziku Pak choi | No difference | Prune peel | No difference |
| Petra (basil) | No difference | Sweet marjoram | No difference | Yuzu seed | No difference |
| Bouquet (dill) | Difference of 7 days or more | Chervil | No difference | Karin seed | No difference |
| Nasturtium | Difference of 3 to 6 days | Chive | No difference | Sicklefruit fenugreek | No difference |
| German chamomile | No difference | Peppermint | No difference | Ginger (herb tea) | Difference of 7 days or more |
| Naga-san (pumpkin) | No difference | Watercress | No difference | Stevia leaf (herb tea) | Difference of less than 3 days |

[Table 2]

| Edible plant | Difference in the number of days until precipitation | Edible plant | Difference in the number of days until precipitation | Edible plant | Difference in the number of days until precipitation |
|---|---|---|---|---|---|
| Dandelion leaf (herb tea) | No difference | Wild herb tea senna tea | No difference | Bok choy | No difference |
| Dandelion root (herb tea) | No difference | Wild herb tea Chinese wolfberry fruit | No difference | Water spinach | No difference |
| Peppermint (herb tea) | Difference of 7 days or more | Komatsuna powder | No difference | Cardamon | Difference of 7 days or more |
| Horsetail (herb tea) | Difference of less than 3 days | Lemon powder | No difference | Korean red pepper | No difference |
| Watercress (raw, aboveground portion) | Difference of less than 3 days | Purple sweet potato powder | No difference | Caraway | Difference of 7 days or more |
| Rape (raw, aboveground portion) | No difference | Lotus root powder | No difference | Mustard seed/ brown | No difference |
| Dropwort (raw, aboveground portion) | No difference | Corn powder | No difference | Mustard seed/ yellow | No difference |
| Nettle (herb tea) | No difference | Yuzu powder | No difference | Mustard/yellow | No difference |
| Mallow blue (herb tea) | No difference | Bamboo peel | No difference | Ginkgo (herb tea) | Difference of 7 days or more |

(continued)

| Edible plant | Difference in the number of days until precipitation | Edible plant | Difference in the number of days until precipitation | Edible plant | Difference in the number of days until precipitation |
|---|---|---|---|---|---|
| Yuzu ichimi | Difference of 3 to 6 days | Hard rush | No difference | Safflower (herb tea) | No difference |
| Ginger (powder) | Difference of 7 days or more | Cherry leaf, green | No difference | Rosemary | No difference |
| Turmeric (powder) | No difference | Cherry life, brown | No difference | Bird's eye | Difference of less than 3 days |
| Poppy sheet | No difference | Veitch's bamboo, domestic, dark green | No difference | Kesshoku | No difference |
| Bee pollen | No difference | Veitch's bamboo, Chinese, green | No difference | *Atractylodes rhizome* | Difference of 7 days or more |
| Vanilla bean (whole) | No difference | Okra powder | No difference | Dong quai | No difference |
| Chinese wolfberry fruit | No difference | Edamame powder | No difference | Astragalus root | No difference |
| Gardenia fruit (whole) | No difference | Horsetail tea | No difference | Byakushi | Difference of less than 3 days |
| Parsley | No difference | Tomato powder | No difference | Dried magnolia leaf | No difference |
| Udo (raw, aboveground | Difference of 7 | Garlic powder | No difference | Radish | No difference |
| portion) | days or more | | | | |
| Coriander (raw, whole grass) | Difference of less than 3 days | Dandelion coffee | No difference | Garland chrysanthemum | No difference |
| Fennel (raw, aboveground portion) | Difference of less than 3 days | Black soybean flour | No difference | Green onion sprout | No difference |
| Sweet basil (freeze-dried) | Difference of 7 days or more | Jew's mallow powder | No difference | Japanese honewort | Difference of 7 days or more |
| Kaffir lime (whole) | No difference | Jerusalem artichoke powder | No difference | Pink radish | No difference |
| Lemon grass (whole) | No difference | Kintoki ginger powder | Difference of 3 to 6 days | Green radish | No difference |
| Orange flower (herb tea) | No difference | Green barley powder | No difference | Vitamin-na | No difference |
| Lavender (herb tea) | Difference of less than 3 days | lees of linseed oil, undried | No difference | Onion | No difference |

(continued)

| Edible plant | Difference in the number of days until precipitation | Edible plant | Difference in the number of days until precipitation | Edible plant | Difference in the number of days until precipitation |
|---|---|---|---|---|---|
| Verbena (herb tea) | No difference | Sweet potato powder | No difference | Ginger mince | Difference of 7 days or more |
| Coconut long | No difference | Cabbage powder | No difference | Tamarind | No difference |
| Blue poppy seed | No difference | Yacon leaf powder | Difference of less than 3 days | Tarragon/ Estragon | Difference of 7 days or more |
| Mango powder | Difference of less than 3 days | Red beefsteak plant powder | Difference of less than 3 days | Citrus unshiu peel | Difference of less than 3 days |
| Ginger powder | Difference of 7 days or more | Ashitaba powder | No difference | Dill weed | Difference of less than 3 days |
| Wild herb tea jack bean | No difference | Ginger powder | Difference of 3 to 6 days | Dill seed | Difference of 7 days or more |
| Wild herb tea field horsetail | No difference | Yacon potato powder | No difference | Dry onion | No difference |
| Wild herb tea mulberry leaf | Difference of 3 to 6 days | Shitake powder | No difference | Nutmeg | Difference of 3 to 6 days |
| Wild herb tea aloe | No difference | Celery powder | Difference of 3 to 6 days | Red jujube | No difference |
| Burdock powder | No difference | Wild turmeric powder | Difference of 7 days or more | Kinshinsai | No difference |
| Sprouted brown rice powder | No difference | Cleavers | No difference | Broccoli powder | No difference |
| Spinach powder | No difference | Hibiscus | Difference of 3 to 6 days | Lemon peel | Difference of 7 days or more |
| Carrot powder | No difference | Skull cap | No difference | Japanese pepper | No difference |
| Pumpkin powder | No difference | Sweet clover | No difference | Purple shallot (red onion) | No difference |
| Wild herb tea persimmon leaf | No difference | Spinach | No difference | Ashitaba powder | Difference of less than 3 days |
| Wild herb tea corn | No difference | Broccoli | No difference | Black rice powder | No difference |
| Wild herb tea five-leaf ginseng | No difference | Superior (lettuce) | No difference | Angelica | No difference |
| Wild herb tea pu-erh tea | No difference | Swiss chard | No difference | German chamomile | Difference of 3 to 6 days |
| Wild herb tea simon tea | No difference | California red (paprika) | No difference | Yam powder | No difference |
| Wild herb tea reishi | No difference | Red chicory | Difference of less than 3 days | Kale powder | No difference |

(continued)

| Edible plant | Difference in the number of days until precipitation | Edible plant | Difference in the number of days until precipitation | Edible plant | Difference in the number of days until precipitation |
|---|---|---|---|---|---|
| Wild herb tea loquat leaf | No difference | Oresh (endive) | No difference | Mulberry leaf powder | No difference |
| Wild herb tea plantain | No difference | Tokyo bekana (mustard greens) | No difference | Gymnema sylvestre powder | No difference |
| Wild herb tea mugwort | No difference | Green romaine | No difference | Dried immature orange powder | No difference |
| Wild herb tea lotus leaf | No difference | Red romaine | No difference | Licorice powder | Difference of 7 days or more |
| Wild herb tea Veitch's bamboo | No difference | Green oak | No difference | | |
| Wild herb tea dokudami | No difference | Red oak | No difference | | |
| Wild herb tea roasted green tea | No difference | Baby purple | No difference | | |
| Wild herb tea buckwheat tea | No difference | Chirimen mustard | Difference of 3 to 6 days | | |

[Test Example 2: Sensory evaluation]

For 19 extracts the antimicrobial action of which was found (the difference from the control was 7 days or more in the number of days until the proliferation) in Test Example 1, whether the extract was suitable as an ingredient in view of fragrance and taste was sensory evaluated using the following evaluation criterion by a trained evaluator. The results are shown in Table 3. In the cell of "portion used" in the table, 1 indicates seed (seed coat) or fruit (fruit coat), 2 indicates leaves, stems, flowers, or trunks, and 3 indicates roots.

Evaluation Criterion

○: Suitable as an ingredient

△: Less suitable as an ingredient

× : Absolutely not suitable as an ingredient

-: Not evaluated

[Table 3]

| Material | Portion used | Fragrance | Taste | Overall rating |
|---|---|---|---|---|
| Bouquet (Dill) | 1 | Δ | - | × |
| Japanese honewort | 1 | Δ | - | × |

(continued)

| Material | Portion used | Fragrance | Taste | Overall rating |
|---|---|---|---|---|
| Soup celery | 1 | Δ | - | × |
| Ginger mince | 3 | ○ | Δ | Δ |
| Tarragon/Estragon | 2 | ○ | Δ | Δ |
| Dill seed | 1 | Δ | - | × |
| Cardamon | 1 | × | - | × |
| Caraway | 1 | Δ | - | × |
| Ginkgo (Herb tea) | 2 | Δ | - | × |
| Ginger (Herb tea) | 3 | ○ | Δ | Δ |
| Peppermint (Herb tea) | 2 | Δ | - | × |
| Ginger (powder) | 3 | ○ | Δ | Δ |
| Udo (raw, aboveground portion) | 2 | Δ | - | × |
| Sweet basil (freeze-dried) | 2 | × | - | × |
| Lemon peel | 1 | Δ | - | Δ |
| Ginger powder | 3 | ○ | Δ | Δ |
| *Atractylodes rhizome* | 2 | Δ | - | × |
| Wild turmeric powder | 3 | Δ | - | × |
| Licorice powder | 3 | ○ | ○ | ○ |

It was estimated from Table 3 that the extract of licorice powder was the most suitable as an ingredient in the overall rating. The extract was added to commercially available low-salt soy sauce (produced by Kikkoman Corporation) to 100 ppm and subjected to sensory evaluation in terms of fragrance and taste by a panel consisting of five trained evaluators. As a result, it was confirmed that the flavor of the low-salt soy sauce was not impaired, and that the extract was suitable as an ingredient.

[Test Example 3: Identification of a substance in licorice powder having antimicrobial action on lactic acid bacteria]

(Preparation of extract)

To 1 kg of licorice powder (NIPPON FUNMATSU YAKUHIN Co., LTD.) was added 5 L of chloroform (KANTO CHEMICAL CO., INC.), and the mixture was stirred at room temperature for 2 hours and then filtered using a folded filter paper (5C). This filtrate was concentrated to dryness using an evaporator to obtain 11 g of an extract.

(Fraction of substance having antimicrobial action)

A column (3 L) was filled with silica gel (Disogel IR-60-40/63A Cat. 1002A). Around 11 g of the above-mentioned obtained extract was dissolved in around 30 ml of chloroform, and the mixture was fractionated into 13 fractions (A to M in order of elution) with the following solvents sequentially while monitoring by TLC analysis at a flow rate of 40 ml/minute and at 220 nm.

1) Hexane (6 L)
2) Hexane:ethyl acetate = 4:1 (10 L)
3) Hexane:ethyl acetate = 3:1 (12 L)
4) Hexane: ethyl acetate = 2:1 (10 L)

(Evaluation of antimicrobial action of fractions)

Each fraction was added to a plate count agar with BCP (Nissui) to 200 μg/mL, 67 μg/mL, 22 μg/mL, or 7

μg/mL. Then, 1 platinum loop of a bacterial suspension of lactic acid bacteria (*Lactobacillus rennini* and *Lactobacillus acidipiscis*, $10^6$ to $10^7$ cfu/mL) was applied. The bacteria were subjected to anaerobic culture at 30°C for 8 days, and the effect of suppressing the proliferation of lactic acid bacteria was evaluated. In the evaluation, the antimicrobial unit of each of the fractions was calculated using the following expression (1), and the ratio of contribution to the antimicrobial action was calculated from the obtained antimicrobial unit of the fraction using the following expression (2). The results are shown in Table 4.

Expression (1): Antimicrobial unit of a fraction = 100/concentration at which the fraction exhibits antimicrobial action × fraction volume

Expression (2): Rate of contribution to antimicrobial action (%) = (antimicrobial unit of the fraction/antimicrobial unit of all fractions) × 100

[Table 4]

| Fraction | Amount of fraction (g) | Active concentration (μg/mL) | Antimicrobial unit of fraction | Contribution rate (%) |
|---|---|---|---|---|
| A | 0.3 | >200 | - | - |
| B | 1.3 | >200 | - | - |
| C | 0.2 | >200 | - | - |
| D | 0.82 | >200 | - | - |
| E | 0.73 | >200 | - | - |
| F | 0.2 | 22 | 0.91 | 14.5 |
| G | 0.31 | 200 | 0.16 | 2.5 |
| H | 0.21 | 200 | 0.11 | 1.7 |
| I | 0.86 | 22 | 3.91 | 62.2 |
| J | 0.33 | 200 | 0.16 | 2.6 |
| K | 0.41 | 200 | 0.2 | 3.3 |
| L | 0.61 | 200 | 0.31 | 4.9 |
| M | 1.06 | 200 | 0.53 | 8.4 |

As clear from Table 4, it was observed that the fractions F and I each had strong antimicrobial action, and it was strongly estimated that the main antimicrobial component of licorice powder was contained especially in the fraction I. Then, the $^{13}C$-NMR spectrum was measured to identify the antimicrobial component contained in the fraction I. Consequently, the antimicrobial component was estimated to be licoricidin, and when the $^{13}C$-NMR spectrum of the antimicrobial component was compared with the $^{13}C$-NMR spectrum of the preparation (produced by ChemFaces), it was confirmed that both match each other. When the antimicrobial component was subjected to TOF (time-of-flight) mass spectrometry, it was confirmed that the antimicrobial component exhibits the peak of $[M+H]^+$ at an m/z of 425, as with licoricidin.

[Test Example 4: Antimicrobial action of licoricidin on lactic acid bacteria in liquid seasoning (1)]

(1) Production of liquid seasonings

(Production of soy sauce)

Commercially available whole soybean soy sauce (produced by Kikkoman Corporation) was electrodialyzed and then concentrated under reduced pressure to obtain soy sauce at a salt concentration of 0% (w/v) and an alcohol concentration of 0% (v/v). Then, salt and/or alcohol was added to the above-mentioned obtained soy sauce, and the mixture was heat-treated to produce soy sauces at salt concentrations and alcohol concentrations shown in the following Table 5. The pHs of the soy sauces were 4.9 to 5.0.

[Table 5]

| Salt concentration (%(w/v)) | Alcohol concentration (%(v/v)) |
|---|---|
| 0 | 0 |
| | 5 |
| | 10 |
| 4 | 0 |
| | 5 |
| | 10 |
| 8 | 0 |
| | 5 |
| | 10 |
| 12 | 0 |
| | 5 |
| | 10 |

(Production of bonito stock)

Bonito stocks was produced using materials shown in the following Table 6. Specifically, components contained in dried bonito was extracted with alcohol and water; sugar, salt, and seasonings were blended with the obtained extract; and the resulting mixture was heated to produce bonito stock. The salt concentration of the obtained bonito stock was 0.2% (w/v), the alcohol concentration was 0.15% (v/v), and the pH was 6.1.

[Table 6]

| Ingredient | Amount blended |
|---|---|
| Dried bonito | 7.5 g |
| Sugar | 3.2 g |
| Salt | 2.0 g |
| Seasonings (amino acids and the like) | 4.0 g |
| Alcohol | 1.5 mL |
| Water | 993 mL |
| Total | 1000 mL |

(Production of sauce)

Sauce was produced using materials shown in the following Table 7. Specifically, Koikuchi soy sauce (produced

by Kikkoman Corporation), granulated sugar, potato starch, and water were blended in amounts shown in following Table 7, and the resulting mixture was heated to produce sauce. The salt concentration of the obtained sauce was 5.3% (w/v), the alcohol concentration was 0.8% (v/v), and the pH was 5.0.

[Table 7]

| Ingredient | Amount blended (g) |
|---|---|
| Koikuchi soy sauce | 354.6 |
| Granulated sugar | 121 |
| Dogtooth violet starch | 20 |
| Water | 504.4 |
| Total | 1000 |

(Production of Japanese seasoned soup stock)

Japanese seasoned soup stock was produced using materials shown in following Table 8. Specifically, the components contained in dried bonito was extracted with water; Koikuchi soy sauce (produced by Kikkoman Corporation), sugar, and salt were blended with the obtained extract; and the resulting mixture was heated to produce Japanese seasoned soup stock. The salt concentration of the obtained Japanese seasoned soup stock was 3% (w/v), the alcohol concentration was 0.4% (v/v), and the pH was 5.5.

[Table 8]

| Ingredient | Amount blended |
|---|---|
| Koikuchi soy sauce | 105 mL |
| Dried bonito | 31.3 g |
| Sugar | 40 g |
| Salt | 13.6 g |
| Water | 888 mL |
| Total | 1000 mL |

(2) Evaluation of antimicrobial action

Licoricidin (produced by ChemFaces) was added to each of the liquid seasonings produced in the above-mentioned (1) to concentrations of 1 ppm, 5 ppm, 10 ppm, 25 ppm, and 50 ppm. Lactic acid bacteria described in the following Table 9 were then added to $10^6$ to $10^7$ cells/mL and cultured at 30°C for 7 days. After the culture, the number of lactic acid bacteria was measured using the GAM agar medium "Nissui". Liquid seasoning to which licoricidin was not added was used as a control group, and the antimicrobial activity was evaluated in comparison with the number of lactic acid bacteria in the control group. The evaluation was performed using the criterion shown below. The results are shown in Tables 10 to 16.

(Evaluation criterion)

A: The bacterial count decreases to less than 10% of the bacterial count of the control group.
B: The bacterial count decreases to 10% or more and less than 50% of the bacterial count of the control group.
C: The bacterial count decreases to 50% or more and less than 100% of the bacterial count of control group.
D: The bacterial count is the same as the bacterial count of the control group.

[Table 9]

| Lactic acid bacteria | Species name | Species culture collection ID |
|---|---|---|
| A | *Lactobacillus rennini* | DSM 20253 |
| B | *Lactobacillus rennini* | DSM 17732 |
| C | *Lactobacillus acidipiscis* | NBRC 102163 |
| D | *Lactobacillus acidipiscis* | NBRC 102164 |

[Table 10]

| Soy sauce; Lactic acid bacteria A | | | | | | |
|---|---|---|---|---|---|---|
| Salt concentration (%(w/v)) | Alcohol concentration (%(v/v)) | Licoricidin concentration (ppm) | | | | |
| | | 1 | 5 | 10 | 25 | 50 |
| 0 | 0 | D | B | B | A | A |
| | 5 | C | B | B | A | A |
| | 10 | C | C | C | A | A |
| 4 | 0 | B | A | A | A | A |
| | 5 | C | A | A | A | A |
| | 10 | A | A | A | A | A |
| 8 | 0 | C | A | A | A | A |
| | 5 | B | A | A | A | A |
| 12 | 0 | D | A | A | A | A |
| | 5 | A | A | A | A | A |
| In soy sauce at a salt concentration of 8% (w/v) or more and an alcohol concentration of 10% (w/v) in control groups, bacteria did not grow. If the Licoricidin concentration was 0.1 ppm, the antimicrobial activity was not found under all the test conditions. | | | | | | |

[Table 11]

| Soy sauce; Lactic acid bacteria B | | | | | | |
|---|---|---|---|---|---|---|
| Salt concentration (%(w/v)) | Alcohol concentration (%(v/v)) | Licoricidin concentration (ppm) | | | | |
| | | 1 | 5 | 10 | 25 | 50 |
| 0 | 0 | B | B | B | A | A |
| 4 | 0 | B | A | A | A | A |
| 8 | 0 | C | A | A | A | A |
| 12 | 0 | A | A | A | A | A |

[Table 12]

| Soy sauce; Lactic acid bacteria C | | | | | | |
|---|---|---|---|---|---|---|
| Salt concentration (%(w/v)) | Alcohol concentration (%(v/v)) | Licoricidin concentration (ppm) | | | | |
| | | 1 | 5 | 10 | 25 | 50 |
| 0 | 0 | C | C | B | A | A |

(continued)

| Soy sauce; Lactic acid bacteria C | | | | | | |
|---|---|---|---|---|---|---|
| Salt concentration (%(w/v)) | Alcohol concentration (%(v/v)) | Licoricidin concentration (ppm) | | | | |
| | | 1 | 5 | 10 | 25 | 50 |
| 4 | 0 | C | A | A | A | A |
| 8 | 0 | D | A | A | A | A |
| 12 | 0 | B | A | A | A | A |

[Table 13]

| Soy sauce; Lactic acid bacteria D | | | | | | |
|---|---|---|---|---|---|---|
| Salt concentration (%(w/v)) | Alcohol concentration (%(v/v)) | Licoricidin concentration (ppm) | | | | |
| | | 1 | 5 | 10 | 25 | 50 |
| 0 | 0 | C | C | B | A | A |
| 4 | 0 | D | A | A | A | A |
| 8 | 0 | C | A | A | A | A |
| 12 | 0 | D | A | A | A | A |

[Table 14]

| Bonito stock | | | | | |
|---|---|---|---|---|---|
| Lactic acid bacteria | Licoricidin concentration (ppm) | | | | |
| | 1 | 5 | 10 | 25 | 50 |
| A | C | A | A | A | A |
| B | C | A | A | A | A |
| C | C | A | A | A | A |
| D | A | A | A | A | A |

[Table 15]

| Sauce | | | | | |
|---|---|---|---|---|---|
| Lactic acid bacteria | Licoricidin concentration (ppm) | | | | |
| | 1 | 5 | 10 | 25 | 50 |
| A | D | D | A | A | A |
| B | D | A | A | A | A |
| C | B | B | A | A | A |
| D | C | C | A | A | A |

[Table 16]

| Japanese seasoned soup stock | | | | | |
|---|---|---|---|---|---|
| Lactic acid bacteria | Licoricidin concentration (ppm) | | | | |
| | 1 | 5 | 10 | 25 | 50 |
| A | D | B | A | A | A |
| B | C | A | A | A | A |
| C | D | A | A | A | A |
| D | D | A | A | A | A |

It was confirmed that licoricidin exhibited antimicrobial activity to various lactic acid bacteria under the conditions of various salt concentration and various alcohol concentrations.

[Test Example 5: Antimicrobial action of licoricidin on lactic acid bacteria in liquid seasoning (2)]

(Production of soy sauce)

Commercially available whole soybean soy sauce (produced by Kikkoman Corporation) was electrodialyzed and then concentrated under reduced pressure to obtain soy sauce at a salt concentration of 0% (w/v) and an alcohol concentration of 0% (v/v). Then, salt and/or alcohol was added to the above-mentioned obtained soy sauce, and hydrochloric acid or an aqueous sodium hydroxide solution was then added to adjust the pH to a desired pH. Germfree purified water was added, and the mixture was then heat-treated to produce soy sauce at the salt concentration, the alcohol concentration, and the pH shown in the following Table 17.

(Evaluation of antimicrobial action)

Licoricidin (produced by ChemFaces) was added to each of the obtained soy sauces to concentrations of 1 ppm, 5 ppm, 10 ppm, 25 ppm, and 50 ppm. Lactic acid bacteria *(Lactobacillus rennini* (DSM 20253)) were then added to $10^6$ to $10^7$ cells/mL and cultured at 30°C for 7 days. After the culture, the number of lactic acid bacteria was measured using the GAM agar medium "Nissui". Soy sauce to which licoricidin was not added was used as a control group, and the antimicrobial activity was evaluated in comparison with the number of lactic acid bacteria in the control group. The evaluation was performed using the same criterion as in Test Example 4. The results are shown in Tables 18 and 19.

[Table 17]

| Salt concentration (%(w/v)) | Alcohol concentration (%(v/v)) | pH |
|---|---|---|
| 0 | 0 | 4.2, 4.6, 5.0, 5.4, 5.8 |
| | 5 | 4.2, 4.6, 5.0, 5.4, 5.8 |
| 4 | 0 | 4.2, 4.6, 5.0, 5.4, 5.8 |
| | 5 | 4.2, 4.6, 5.0, 5.4, 5.8 |

[Table 18]

| Salt concentration 0% (w/v) | | | | | | |
|---|---|---|---|---|---|---|
| pH | Alcohol concentration (%(v/v)) | Licoricidin concentration (ppm) | | | | |
| | | 1 | 5 | 10 | 25 | 50 |
| 4.2 | 0 | B | B | A | A | A |
| | 5 | B | A | A | A | A |
| 4.6 | 0 | C | B | A | A | A |
| | 5 | C | A | A | A | A |

(continued)

| Salt concentration 0% (w/v) | | | | | | |
|---|---|---|---|---|---|---|
| pH | Alcohol concentration (%(v/v)) | Licoricidin concentration (ppm) | | | | |
| | | 1 | 5 | 10 | 25 | 50 |
| 5.0 | 0 | D | B | B | A | A |
| | 5 | C | B | B | A | A |
| 5.4 | 0 | D | D | C | C | C |
| | 5 | B | A | A | A | A |
| 5.8 | 0 | D | D | D | D | D |
| | 5 | B | A | A | A | A |

[Table 19]

| Salt concentration 4% (w/v) | | | | | | |
|---|---|---|---|---|---|---|
| pH | Alcohol concentration (%(v/v)) | Licoricidin concentration (ppm) | | | | |
| | | 1 | 5 | 10 | 25 | 50 |
| 4.2 | 0 | B | A | A | A | A |
| | 5 | C | A | A | A | A |
| 4.6 | 0 | D | D | A | A | A |
| | 5 | B | A | A | A | A |
| 5.0 | 0 | B | A | A | A | A |
| | 5 | C | A | A | A | A |
| 5.4 | 0 | B | A | A | A | A |
| | 5 | C | A | A | A | A |
| 5.8 | 0 | D | A | A | A | A |
| | 5 | D | D | A | A | A |

[Test Example 6: Antimicrobial action of oxygen-containing heterocyclic compounds on lactic acid bacteria in liquid seasoning (1)]

(Production of soy sauce)

Commercially available whole soybean soy sauce (produced by Kikkoman Corporation) was electrodialyzed and then concentrated under reduced pressure to produce soy sauce at a salt concentration of 0% (w/v) and an alcohol concentration of 0% (v/v). Then, salt and/or alcohol was added to the above-mentioned obtained soy sauce, and the mixture was then heat-treated to produce soy sauce at a salt concentration of 4% (w/v) and an alcohol concentration of 5% (v/v).

(Evaluation of antimicrobial action)

The test substance described in Table 20 was added to the above-mentioned produced soy sauce to concentrations of 1 to 100 ppm. Lactic acid bacteria *(Lactobacillus rennini* (DSM 20253)) were then added to $10^6$ to $10^7$ cells/mL and cultured at 30°C for 7 days. After the culture, the number of lactic acid bacteria was measured using the GAM agar medium "Nissui". Soy sauce to which licoricidin was not added was used as a control group, and the antimicrobial activity was evaluated in comparison with the number of lactic acid bacteria in the control group. The evaluation was performed using the criterion shown below. The results are shown in Table 20.

(Evaluation criterion)

A: The bacterial count decreases to less than 10% of the bacterial count of the control group.
B: The bacterial count decreases to 10% or more and less than 50% of the bacterial count of control group.
C: The bacterial count decreases to 50% or more and less than 100% of the bacterial count of the control group.
D: The bacterial count is the same as the bacterial count of the control group.
-: Not evaluated

[Table 20]

| Test substance (manufacturer) | Test substance concentration (ppm) | | | | | |
|---|---|---|---|---|---|---|
| | 1 | 5 | 10 | 25 | 50 | 100 |
| Licoricidin (ChemFaces) | D | A | A | A | A | - |
| Gancaonin I (ChemFaces) | C | A | A | A | A | A |
| 8-(γ,γ-dimethylallyl)-wighteone (ALB Technology Limited) | C | A | A | A | A | A |
| Glycycoumarin (ChemFaces) | D | B | B | A | A | A |
| Glyasperin C (ChemFaces) | B | B | B | A | A | A |
| Glycyrin (ChemFaces) | D | B | B | A | A | A |
| Isoangustone A (ChemFaces) | B | A | A | A | A | A |
| Licoarylcoumarin (ChemFaces) | D | C | C | B | B | A |

It was confirmed that the oxygen-containing heterocyclic compounds other than licoricidin had antimicrobial activity, as with licoricidin.

[Test Example 7: Antimicrobial action of oxygen-containing heterocyclic compounds on lactic acid bacteria in liquid seasoning (2)]

(Evaluation of antimicrobial action)

The test substance described in Table 21 was added to the soy sauce produced in Test Example 6 to concentrations of 0.1 ppm, 0.5 ppm, 1 ppm, 5 ppm, 25 ppm, and 50 ppm. Lactic acid bacteria (*Lactobacillus fructivores* (NBRC 13954)) were then added to $10^4$ to $10^5$ cells/mL and cultured at 30°C for 7 days. After the culture, the number of lactic acid bacteria was measured using Difco Lactobacilli MRS Agar. Soy sauce to which the test substance was not added was used as a control group, and the antimicrobial activity was evaluated in comparison with the number of lactic acid bacteria in the control group. The evaluation was performed using the following criterion. The results are shown in Table 21.

(Evaluation criterion)

A: The bacterial count decreases to less than 10% of the bacterial count of a control group.
B: The bacterial count decreases to 10% or more and less than 50% of the bacterial count of the control group.
C: The bacterial count decreases to 50% or more and less than 100% of the bacterial count of the control group.
D: The bacterial count is the same as the bacterial count of the control group.

[Table 21]

| Test substance (manufacturer) | Test substance concentration (ppm) | | | | | |
|---|---|---|---|---|---|---|
| | 0.1 | 0.5 | 1 | 5 | 25 | 50 |
| Licoricidin (ChemFaces) | B | B | B | B | A | A |
| Gancaonin I (ChemFaces) | B | C | B | C | B | A |
| 8-($\gamma$,$\gamma$-dimethylallyl)-wighteone (ALB Technology Limited) | B | B | B | A | A | A |
| Glycycoumarin (ChemFaces) | B | B | B | B | A | A |
| Glyasperin C (ChemFaces) | B | B | B | B | B | A |
| Glycyrin (ChemFaces) | B | B | B | B | B | B |
| Isoangustone A (ChemFaces) | B | B | C | B | A | A |
| Licoarylcoumarin (ChemFaces) | B | B | B | C | B | B |

It was confirmed that the oxygen-containing heterocyclic compounds other than licoricidin had antimicrobial activity, as with licoricidin.

[Test Example 8: Antimicrobial action of licoricidin on flat sour bacteria in liquid seasoning]

(Production of Japanese seasoned soup stock)

Japanese seasoned soup stock was produced by using materials shown in the following Table 22. Specifically, components contained in dried bonito was extracted with water; Koikuchi soy sauce (produced by Kikkoman Corporation), sugar, and salt were blended with the obtained extract; and the resulting mixture was heated to produce Japanese seasoned soup stock. The salt concentration of the obtained Japanese seasoned soup stock was 3% (w/v), the alcohol concentration was 0.4% (v/v), and the pH was 5.2.

[Table 22]

| Ingredient | Amount blended |
|---|---|
| Koikuchi soy sauce | 105 mL |
| Dried bonito | 31.3 g |
| Sugar | 40 g |
| Salt | 13.6 g |
| Water | 888 mL |
| Total | 1000 mL |

(Evaluation of antimicrobial action)

Licoricidin (produced by ChemFaces) was added to the produced Japanese seasoned soup stock to concentrations of 1 ppm, 5 ppm, 10 ppm, 25 ppm, and 50 ppm. Flat sour bacteria *(Bacillus coagulans* (IFO12714)) were then added to $10^4$ to $10^5$ cells/mL and cultured at 45°C for 7 days. Japanese seasoned soup stock to which licoricidin was not added was used as a control, and the antimicrobial activity was evaluated by comparing the pHs. The evaluation was performed using the following criterion. The results are shown in Table 23.

(Evaluation criterion)

A: There is no change in pH as compared with the control.
B: The pH decreases as compared with the control.

[Table 23]

| Licoricidin (ChemFaces) (ppm) | 0 (Control) | 1 | 5 | 10 | 25 | 50 |
|---|---|---|---|---|---|---|
| pH | 4.1 | 4,1 | 4.1 | 5.3 | 5.3 | 5.2 |
| Evaluation | - | B | B | A | A | A |

It was confirmed that licoricidin exhibited antimicrobial activity to flat sour bacteria.

[Test Example 9: Antimicrobial action of licoricidin on lactic acid bacteria in liquid seasonings and food (3)]

(1) Production of liquid seasonings and food

(Production of Liquid dressing)

Liquid dressing was produced by using materials shown in the following Table 24. Specifically, Koikuchi soy sauce (produced by Kikkoman Corporation), table vinegar, mirin, granulated sugar, kelp stock, and water were blended in amounts shown in the following Table 24, and the resulting mixture was heated to produce liquid dressing. The salt concentration of the obtained liquid dressing was 2.6% (w/v), the alcohol concentration was 0.8% (v/v), the pH was 4.2.

[Table 24]

| Ingredient | Amount blended (g) |
|---|---|
| Koikuchi soy sauce | 18.72 |
| Table vinegar | 2.54 |
| Mirin | 2.9 |
| Granulated sugar | 5 |
| Kelp stock | 16 |
| Water | 54.84 |
| Total | 100 |

(Production of lightly-pickled vegetables)

Seasoning liquid for lightly-pickled vegetables were produced by using materials shown in the following Table 25. Specifically, Usukuchi soy sauce (produced by Kikkoman Corporation), table vinegar, corn syrup, isomerized sugar, salt, granulated sugar, sodium glutamate (MSG), lemon fruit juice, kelp stock, and water were blended in amounts shown in the following Table 25, and the mixture was heated to produce seasoning liquid for lightly-pickled vegetables. Chinese cabbages were fully soaked therein to produce lightly-pickled vegetables. The salt concentration of the obtained seasoning liquid for lightly-pickled vegetables was 3.1% (w/v), the alcohol concentration was 0.01% (v/v), and the pH was 4.4.

[Table 25]

| Ingredient | Amount blended |
|---|---|
| Usukuchi soy sauce | 0.3 mL |
| Table vinegar | 0.5 mL |
| Corn syrup | 15 g |
| Isomerized sugar | 2 g |
| Salt | 3 g |
| Granulated sugar | 4 g |
| MSG | 0.4 g |
| Lemon fruit juice | 0.68 g |

(continued)

| Ingredient | Amount blended |
|---|---|
| Kelp stock | 3.2 mL |
| Water | 83.45 mL |
| Total | 100 mL |

(Production of cooking vinegar)

Cooking vinegar was produced by using materials shown in the following Table 26. Specifically, table vinegar, salt, granulated sugar, lemon fruit juice, sodium glutamate (MSG), and water in amounts shown in following Table 26 were blended, and the mixture was heated to produce cooking vinegar. The salt concentration of the obtained cooking vinegar was 1.5% (w/v), the alcohol concentration was 0% (v/v), and the pH was 3.8.

[Table 26]

| Ingredient | Amount blended |
|---|---|
| Table vinegar | 0.1 mL |
| Salt | 1.5 g |
| Granulated sugar | 10 g |
| Lemon fruit juice | 5.1 g |
| MSG | 0.4 g |
| Water | 87.3 mL |
| Total | 100 mL |

(2) Evaluation of antimicrobial action

Licoricidin (produced by ChemFaces) was added to each of the above-mentioned produced liquid dressing, lightly-pickled vegetables, and cooking vinegar to concentrations of 1 ppm, 5 ppm, 10 ppm, 25 ppm, and 50 ppm. Lactic acid bacteria *(Lactobacillus rennini* (DSM 20253)) were then added to $10^6$ to $10^7$ cells/mL. The bacteria were cultured in the liquid dressing and the lightly-pickled vegetables at 30°C for 7 days, and the bacteria were cultured in the cooking vinegar at 30°C for 3 days. After the culture, the number of lactic acid bacteria was measured using the GAM agar medium "Nissui". The liquid dressing, the lightly-pickled vegetables, and the cooking vinegar to which licoricidin was not added were used as respective control groups, and the antimicrobial activity was evaluated in comparison with the number of lactic acid bacteria in the control group. The evaluation was performed using the same criterion as in Test Example 4. The results are shown in Table 27.

(Evaluation criterion)

A: The bacterial count decreases to less than 10% of the bacterial count of the control group.
B: The bacterial count decreases to 10% or more and less than 50% of the bacterial count of the control group.
C: The bacterial count decreases to 50% or more and less than 100% of the bacterial count of control group.
D: The bacterial count is the same as the bacterial count of the control group.

[Table 27]

| Evaluation sample | Licoricidin concentration (ppm) | | | | |
|---|---|---|---|---|---|
| | 1 | 5 | 10 | 25 | 50 |
| Liquid dressing | A | A | A | A | A |

(continued)

| Evaluation sample | Licoricidin concentration (ppm) | | | | |
|---|---|---|---|---|---|
| | 1 | 5 | 10 | 25 | 50 |
| Lightly-pickled vegetables | B | C | A | A | A |
| Cooking vinegar | A | A | A | A | A |

It was confirmed that licoricidin exhibited antimicrobial activity to lactic acid bacteria in the various liquid seasonings and the food.

**Claims**

1. A food/beverage article comprising:

one or more compounds represented by formula (I):

[Chemical Formula 1]

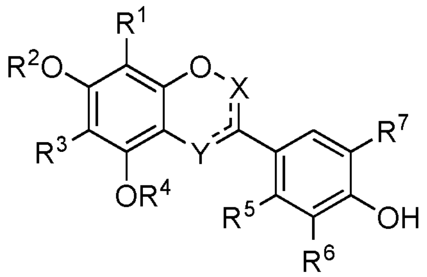

(I)

wherein, $R^1$ and $R^3$ each independently represent a hydrogen atom or a $C_{2-6}$ alkenyl group, $R^2$ and $R^4$ each independently represent a hydrogen atom or a $C_{1-4}$ alkyl group, $R^5$ and $R^7$ each independently represent a hydrogen atom or a hydroxyl group, $R^6$ represents a hydrogen atom or a $C_{2-6}$ alkenyl group, X represents a direct bond, $-CH_2-$, -CH=, or -C(=O)-, Y represents $-CH_2-$, -CH=, or -C(=O)-, and a bond represented by the following formula:

[Chemical Formula 2] 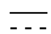

represents a single bond or a double bond,
wherein a total of concentrations of the compounds is 1 ppm or more.

2. The food/beverage article according to claim 1, wherein the compound is at least one selected from the group consisting of licoricidin, gancaonin I, 8-(γ,γ-dimethylallyl)-wighteone, glycycoumarin, glyasperin C, glycyrin, isoangustone A, and licoarylcoumarin.

3. The food/beverage article according to claim 1 or 2, wherein the food/beverage article is liquid seasoning or food.

4. The food/beverage article according to claim 3, wherein the liquid seasoning is soy sauce, soup stock, Japanese seasoned soup stock, sauce, dressing, or cooking vinegar, and the food is lightly-pickled vegetables.

5. An antimicrobial agent to lactic acid bacteria and/or the flat sour bacteria comprising, as an active ingredient, a compound represented by formula (I):

[Chemical Formula 3]

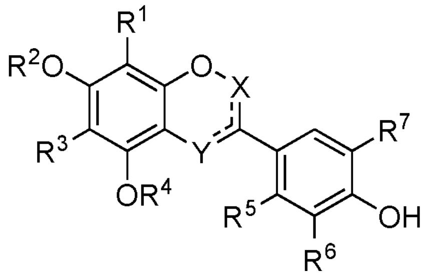

(I)

wherein, $R^1$ and $R^3$ each independently represent a hydrogen atom or a $C_{2-6}$ alkenyl group, $R^2$ and $R^4$ each independently represent a hydrogen atom or a $C_{1-4}$ alkyl group, $R^5$ and $R^7$ each independently represent a hydrogen atom or a hydroxyl group, $R^6$ represents a hydrogen atom or a $C_{2-6}$ alkenyl group, X represents a direct bond, $-CH_2-$, -CH=, or -C(=O)-, Y represents $-CH_2-$, -CH=, or -C(=O)-, and a bond represented by the following formula:

[Chemical Formula 4] 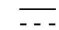

represents a single bond or a double bond.

**6.** The antimicrobial agent according to claim 5, wherein the compound is licoricidin, gancaonin I, 8-(γ,γ-dimethylallyl)-wighteone, glycycoumarin, glyasperin C, glycyrin, isoangustone A, or licoarylcoumarin.

**7.** A method for suppressing proliferation of lactic acid bacteria and/or flat sour bacteria in a food/beverage article, comprising: adjusting a total of concentrations of compounds represented by formula (I) in the food/beverage article to 1 ppm or more:

[Chemical Formula 5]

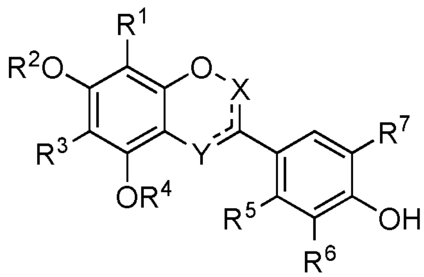

(I)

wherein, $R^1$ and $R^3$ each independently represent a hydrogen atom or a $C_{2-6}$ alkenyl group, $R^2$ and $R^4$ each independently represent a hydrogen atom or a $C_{1-4}$ alkyl group, $R^5$ and $R^7$ each independently represent a hydrogen atom or a hydroxyl group, $R^6$ represents a hydrogen atom or a $C_{2-6}$ alkenyl group, X represents a direct bond, $-CH_2-$, -CH=, or -C(=O)-, Y represents $-CH_2-$, -CH=, or -C(=O)-, and a bond represented by the following formula:

[Chemical Formula 6] 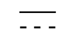

represents a single bond or a double bond.

**8.** The method according to claim 7, wherein the compound is licoricidin, gancaonin I, 8-(γ,γ-dimethylallyl)-wighteone, glycycoumarin, glyasperin C, glycyrin, isoangustone A, or licoarylcoumarin.

## INTERNATIONAL SEARCH REPORT

International application No.
PCT/JP2021/028606

**A. CLASSIFICATION OF SUBJECT MATTER**

Int. Cl. A23L3/3544(2006.01)i, A61P31/00(2006.01)i, A23L27/00(2016.01)i, A23L27/50(2016.01)i, A01P3/00(2006.01)i, A01N43/16(2006.01)i, A61K31/343(2006.01)i, A61K31/352(2006.01)i, A61K31/353(2006.01)i, A61K31/37(2006.01)i
FI: A23L3/3544, A61K31/343, A61K31/37, A61K31/352, A61K31/353, A61P31/00, A23L27/50 105, A23L27/00 D, A01N43/16 C, A01P3/00

According to International Patent Classification (IPC) or to both national classification and IPC

**B. FIELDS SEARCHED**

Minimum documentation searched (classification system followed by classification symbols)
Int. Cl. A23L3/3544, A61P31/00, A23L27/00, A23L27/50, A01P3/00, A01N43/16, A61K31/343, A61K31/352, A61K31/353, A61K31/37

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | 「サンリコリスA」のカタログ. 2017, in particular, "Characteristics", non-official translation ("Sain Licorice A" Catalog.) | 1-8 |
| X | 北条寛. 甘草油性抽出物製剤「サンリコリス」の抗菌活性. 月刊フードケミカル. 2003, vol. 19, no. 6, pp. 116-122, in particular, "3. Licorice Oil Extract Preparation 'Sain Licorice'", tables 3-8, non-official translation (HOJO, Hiroshi. Antibacterial Activity of Licorice Oil Extract Preparation "Sain Licorice". Monthly Food Chemical.) | 1-8 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16.08.2021 | 14.09.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/028606

C (Continuation). DOCUMENTS CONSIDERED TO BE RELEVANT

| Category* | Citation of document, with indication, where appropriate, of the relevant passages | Relevant to claim No. |
|---|---|---|
| X | 吉川展司 et al. 甘草およびその成分(グリチルリチン酸等)について. FFIジャーナル. 2012, vol. 217, no. 1, pp. 38-46, in particular, fig. 2, "3-4. Use as Food, Food Additive", (FURUKAWA, Hiroshi, ITO, Makoto. Licorice and Its Ingredients (Glycyrrhizinic Acid, etc.). Foods & Food Ingredients Journal of Japan.) | 1-8 |
| X | JP 63-218670 A (MARUZEN KASEI CO., LTD.) 12 September 1988 (1988-09-12), claims, p. 2, upper right column, line 13 to lower right column, line 4, examples | 1-8 |
| X | JP 2-204417 A (MARUZEN KASEI CO., LTD.) 14 August 1990 (1990-08-14), claims, p. 1, left column, lines 9-13, examples | 1-8 |
| X | WO 2003/070263 A1 (KANEKA CORP.) 28 August 2003 (2003-08-28), claims, p. 12, line 25 to p. 14, line 3, examples | 1-8 |
| X | WO 2003/084556 A1 (KANEKA CORP.) 16 October 2003 (2003-10-16), claims, p. 7, line 1 to p. 8, line 12, examples | 1-8 |
| X | WO 2007/123044 A1 (KANEKA CORP.) 01 November 2007 (2007-11-01), claims, paragraphs [0015], [0025], examples | 1-8 |
| X | WO 2008/143182 A1 (KANEKA CORP.) 27 November 2008 (2008-11-27), claims, paragraphs [0006], [0013], [0051]-[0058], examples | 1-8 |
| X | JP 2019-066291 A (MARUZEN KASEI CO., LTD.) 25 April 2019 (2019-04-25), claims, paragraph [0026], examples | 1-8 |
| X | JP 2006-045121 A (MARUZEN KASEI CO., LTD.) 16 February 2006 (2006-02-16), claims, paragraphs [0028]-[0032], examples | 1-8 |
| X | JP 59-046210 A (MARUZEN KASEI CO., LTD.) 15 March 1984 (1984-03-15), claims, examples | 1-8 |

Form PCT/ISA/210 (continuation of second sheet) (January 2015)

**INTERNATIONAL SEARCH REPORT**

International application No.
PCT/JP2021/028606

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The composition containing the compound represented by general formula (1), which is the feature shared among the inventions as in claims 1, 5, and 7, does not make a contribution over the prior art in light of the content disclosed in documents 1-12, and thus this feature cannot be said to be a special technical feature.

Therefore, there are no identical or corresponding special technical features among the inventions as in claims 1, 5, and 7.

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2021/028606

| Patent Documents referred to in the Report | Publication Date | Patent Family | Publication Date |
|---|---|---|---|
| JP 63-218670 A | 12.09.1988 | (Family: none) | |
| JP 2-204417 A | 14.08.1990 | (Family: none) | |
| WO 2003/070263 A1 | 28.08.2003 | US 2005/0118288 A1<br>claims, examples<br>EP 1477177 A1 | |
| WO 2003/084556 A1 | 16.10.2003 | US 2005/0118289 A1<br>claims, examples<br>EP 1491204 A1 | |
| WO 2007/123044 A1 | 01.11.2007 | US 2009/0087419 A1<br>claims, examples<br>EP 2018869 A1 | |
| WO 2008/143182 A1 | 27.11.2008 | US 2008/0286254 A1<br>claims, examples<br>EP 2163252 A1 | |
| JP 2019-066291 A | 25.04.2019 | (Family: none) | |
| JP 2006-045121 A | 16.02.2006 | (Family: none) | |
| JP 59-046210 A | 15.03.1984 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *JOURNAL OF THE BREWING SOCIETY OF JAPAN,* 2008, vol. 103 (2), 94-99
- B. coagulans. Fundamentals of Food Microbiology. KODANSHA LTD, 2015, 15